# EUROPEAN PATENT SPECIFICATION

(11) **EP 2 368 564 B1**
(45) Date of publication and mention of the grant of the patent: **02.09.2015**
(21) Application number: 10002990.9
(22) Date of filing: 22.03.2010
(51) Int. Cl.: A61K 38/17, A61P 37/00

(54) **Pharmaceutical composition containing SAA for use in the treatment of acute and chronic dysregulated inflammatory diseases or conditions**
Pharmazeutische Zusammensetzung enthaltend SAA zur Verwendung in der Behandlung von akuten und chronischen dysregulierten Entzündungskrankheiten oder -leiden in der akuten Phase
Composition pharmaceutique comprenant de SAA et son utilisation dans le traitement des maladies ou conditions inflammatoires dérégulées aiguës et chroniques

(43) Date of publication of application: 28.09.2011
(60) Divisional application: 15177622.6
(73) Proprietor: Linke, Reinhold Paul, 81475 München (DE)
(72) Inventor: Sander, Leif Erik, New York 10128- NY (US); Trautwein, Christian, 52066 Aachen (DE); Linke, Reinhold Paul, 81475 München (DE)
(74) Representative: Seifert, Ruth

(56) References cited:
- US-A1- 2008 167 242
- SANDER LEIF E ET AL: "Hepatic acute-phase proteins control innate immune responses during infection by promoting myeloid-derived suppressor cell function." THE JOURNAL OF EXPERIMENTAL MEDICINE 5 JUL 2010 LNKD- PUBMED:20530204, vol. 207, no. 7, 5 July 2010 (2010-07-05), pages 1453-1464, XP002595841 ISSN: 1540-9538
- KLEIN CHRISTIAN ET AL: "The IL-6-gp130-STAT3 pathway in hepatocytes triggers liver protection in T cell-mediated liver injury." THE JOURNAL OF CLINICAL INVESTIGATION APR 2005 LNKD- PUBMED:15761498, vol. 115, no. 4, April 2005 (2005-04), pages 860-869, XP002595842 ISSN: 0021-9738
- DELANO MATTHEW J ET AL: "MyD88-dependent expansion of an immature GR-1(+)CD11b(+) population induces T cell suppression and Th2 polarization in sepsis." THE JOURNAL OF EXPERIMENTAL MEDICINE 11 JUN 2007 LNKD- PUBMED:17548519, vol. 204, no. 6, 11 June 2007 (2007-06-11), pages 1463-1474, XP002595843 ISSN: 0022-1007
- HAILE L A ET AL: "Myeloid-Derived Suppressor Cells in Inflammatory Bowel Disease: A New Immunoregulatory Pathway" GASTROENTEROLOGY, ELSEVIER, PHILADELPHIA, PA LNKD- DOI:10.1053/J.GASTRO.2008.06.032, vol. 135, no. 3, 1 September 2008 (2008-09-01), pages 871-881.E5, XP025641364 ISSN: 0016-5085 [retrieved on 2008-06-12]
- GABRILOVICH D I ET AL: "Myeloid-derived suppressor cells as regulators of the immune system" NATURE REVIEWS. IMMUNOLOGY, NATURE PUBLISHING GROUP, GB LNKD- DOI:10.1038/NRI2506, vol. 9, no. 3, 1 March 2009 (2009-03-01), pages 162-174, XP002588070 ISSN: 1474-1733 [retrieved on 2009-02-06]
- PARRISH WILLIAM R ET AL: "Experimental therapeutic strategies for severe sepsis: mediators and mechanisms." ANNALS OF THE NEW YORK ACADEMY OF SCIENCES NOV 2008 LNKD- PUBMED:19076379, vol. 1144, November 2008 (2008-11), pages 210-236, XP9137394 ISSN: 1749-6632
- SANDER L ET AL: "763 THE HEPATIC ACUTE PHASE RESPONSE CONTROLS INFLAMMATION BY PROMOTING MYELOID DERIVED SUPPRESSOR CELL FUNCTIONS IN SEPSIS", JOURNAL OF HEPATOLOGY, ELSEVIER, AMSTERDAM, NL, vol. 50, 1 April 2009 (2009-04-01), page S280, XP026496376, ISSN: 0168-8278, DOI: 10.1016/S0168-8278(09)60765-0 [retrieved on 2009-04-01]
- L. E. Sander ET AL: "Hepatic acute-phase proteins control innate immune responses during infection by promoting myeloid-derived suppressor cell function", Journal of Clinical Investigation, vol. 100, no. 3, 5 July 2010 (2010-07-05), pages 522-1464, XP055168824, ISSN: 0021-9738, DOI: 10.1016/j.ccr.2007.12.004

## Description

The present invention describes a novel intervention strategy for the treatment of dysregulated inflammations by using hepatic acute phase proteins serum amyloid A (SAA). The said protein is to be used as pharmaceutical compositions in the treatment of conditions and diseases characterized by dysregulated inflammations. Besides direct anti-inflammatory as well as host-defensive effects, a hitherto unrecognized regulation of myeloid-derived suppressor cells (MDSC) is uncovered. Myeloid derived suppressor cells seem to be key regulators of inflammatory responses during sepsis and thus represent a valuable target for treatment of these disorders. In a further aspect, the present invention concerns the use of an effective amount of the above identified agent for treatment of sepsis.

### BACKGROUND AND STATE OF THE ART

Dysregulated inflammation is a common culprit of various medical conditions ranging from autoinflammatory diseases like IBD or rheumatoid arthritis (RA) to (polytraumatic) injury and sepsis.

### Acute inflammatory syndromes and conditions

Sepsis is a major cause of mortality worldwide characterised by an uncontrolled inflammatory response to infections. Unfortunately, therapeutic strategies targeting pro-inflammatory cytokines have had only very limited success, demonstrating the complex pathogenesis of disorders that are determined by a variety of both pathogens and host factors.

Uncontrolled activation of the immune system can lead to a systemic inflammatory response syndrome (SIRS). SIRS is most frequently caused by infections in the course of sepsis; non-infectious causes of SIRS include trauma, burns, pancreatitis, ischemia and haemorrhage. Common complications of SIRS include failure of one or more organs or organ systems, e.g. acute kidney injury, acute lung injury like acute respiratory distress syndrome (ARDS), or multiple organ dysfunction syndrome (MODS).

A major complication of sepsis and SIRS is the development of septic shock and subsequent failure of multiple organ systems (MODS) or multiple organ failure syndrome (MOFS) and it is associated with a poor outcome and high mortality. In fact, sepsis and its complications represent the main cause of mortality of intensive care patients. Mortality rate of 20% of patients with severe sepsis and 46% of patients with septic shock has been reported.

A definition of SIRS, sepsis and organ failure etc. as well as guidelines for the use of innovative therapies in sepsis are outlined in Bone, R.C. et.al., Chest 1992, 101, 1644-1655. Further, this document provides guidelines for determining sepsis etc. based on different parameters like temperature, heart rate etc.

Treatment of sepsis and SIRS is mainly based on administration of antibiotics, fluid replacement, surgical drainage of infective fluid collections, and appropriate support for organ dysfunction. However, dysregulated inflammatory responses rather than the infectious agent or the trauma itself is often the cause of severe complications that are not sufficiently targeted by the general therapeutic strategies outlined above. Thus, significant efforts have been made to inhibit key mediators of inflammation, yet most studies yielded rather discouraging results. Hence, there is an urgent need for alternative anti-inflammatory strategies.

### Chronic inflammatory conditions

Diseases characterized by chronic inflammation include autoinflammatory and autoimmune diseases like inflammatory bowel disease (IBD), RA (rheumatoid arthritis) or psoriasis. Here chronic activation of immune cells drives tissue destruction, which can proceed to a loss of organ functions and severe complications such as intestinal strictures, joint destructions and malformations. In addition chronic inflammatory organ damage is considered a major factor contributing to cancer development and liver cirrhosis.

### Acute Phase Proteins

Acute phase proteins (APPs) are an evolutionarily conserved family of proteins produced mainly in the liver in response to infections and inflammations. They are defined as proteins whose serum levels change by more than 25 % during inflammation. Some APPs increase (positive APPs) and others decrease (negative APPs) The positive APPs (in the following referred to as APPs) are mainly synthesized in the liver and are regarded as important components of the innate immune response to infection. Many APPs are known as potent opsonins and activators of innate immune cells like neutrophils, but they also have some anti-inflammatory properties. Due to the large diversity of APPs with pro- and anti-inflammatory function their overall role during infections is still not well defined.

Interleukin-6 (IL-6) is one of the major inducer of APP production in hepatocytes in vitro. IL-6 shares the common signaling receptor gp130 with eight other cytokines. Innate immune cell activation is critical for host defence against invading microorganisms and for the subsequent generation of an adaptive immune response. On the other hand, pro-inflammatory mediators produced by innate immune cells are considered key elements in the pathogenesis of severe sepsis and multi-organ failure. A complex network of activating and inhibiting functions leading to regulatory pathways controls innate immune responses. It is known that IL-6 represents also one of the major pro-inflammatory mediators and of sepsis is a strong stimulator of APP production in the liver in vivo. Notably, patients with chronic liver diseases have a significantly increased risk of acquiring sepsis and its accompanying complications, and, thus, have higher sepsis related mortality. Septic patients with chronic liver disease exhibit prolonged exacerbated inflammation and elevated levels of inflammatory cytokines.

As mentioned above, APPs form a large group of proteins with very diverse functions. APPs include molecules such as CRP (C-reactive protein) and SAA (serum amyloid A).

CRP is a commonly used clinical acute phase response and APP-marker in patients. Its serum levels have been shown to correlate well with systemic or local inflammatory responses. CRP has been shown to bind phosphocholine residues expressed on the surface of dead or dying cells as well as on some bacteria, which results in the activation of the complement system. Complement activation constitutes a major innate defense mechanisms against bacterial invaders. CRP is produced in the liver by hepatocytes in response to gp130-activation by IL-6 or other IL-6 family cytokines.

Serum amyloid A protein (SAA) constitutes a highly conserved multi-gene apolipoprotein family consisting of four genes in humans of which only three are expressed with SAA3 being a pseudogene. SAA1 and SAA2 comprise the acute phase SAAs (A-SAA) which are chemically closely related with less than 10 % different of the amino acids sequence while SAA4 differs by 47% and 45% from SAA1 and SAA2, respectively. In addition, A-SAA-allotypes (SAA1.1-5 and SAA2.1-2) are known. Since SAA4 does not show the conspicuous acute phase behaviour it is called constitutive SAA (C-SAA) and its function is largely unknown. The A-SAAs have 104 amino acids while C-SAA has 112 amino acids (NM 199161). The larger size of the latter is due to an insertion of an octapeptide between residue 69 and 70 whose function is unknown. All SAA molecules in all species analyzed so far share an invariant peptide at position 33-43 which has been shown to convey major SAA-functions by being blocked through monoclonal antibodies directed against this peptide. In addition within this invariant peptide the tripeptide RGN has been noticed. It resembles the RGD cell-binding motive of fibronectin which is one of the cell adhesive proteins within the extracellular matrix and which can also permit neoplastic cells to home and build metastases in the connective tissue displaying these cell binding motives In addition, the peptide YIGSD (Seq. ID. No. 1) at position 29-33 only on SAA1 resembles the cell adhesive motive YIGSR (Seq. ID. No. 2) of the extracellular matrix protein laminin. Both motives on SAA can counteract the homing of metastatic cells by competing with similar motives on fibrinectin and laminin (as examples of families of cells having similar functions) in the various connective tissues. SAA can also become pathogenic afte various long lasting acute phase reactions by the polymerization to amyloid of the SAA1/2 fragment1-76 which causes the fatal disease AA amyloidosis.

SAA has been associated with a number of chronic and acute inflammatory conditions regardless of origin. In response to acute inflammatory stimuli, which are in particular represented by the monocyte-derived IL-1, IL6 and TNFα, plasma levels of SAA1 and SAA2 can rapidly increase more than 1,000 fold within 6 to 12 hours.

CXCL1 (NM 001511, Seq ID. No.3) is a small cytokine belonging to the Cxc chemokine family. CXCL1 plays a role in spinal cord development and is involved in the processes of angiogenesis, inflammation, wound healing and tumorigenesis. It is mainly known for its role in recruiting polymorphonuclear leukocytes to the site of inflammation through its direct chemotactic activity. CXCL1 exerts its functions through the chemokine receptor CXCR2.

Interestingly, it has been shown that both proteins, SAA2 and KC, are potent inhibitors of Con A-induced hepatitis (Klein et al., 2005).

Furthermore, it has been shown that the administration of SAA and KC reversed the detrimental effects of gp130 deficiency and restored mobilization, survival and splenic accumulation of MDSCs (Sander et al., 2009).

Given the large number and vast diversity of APPs with both pro- and anti-inflammatory functions, their overall role during dysregulated inflammatory diseases e.g. sepsis, remains still ill defined.

### Myeloid Derived Suppressor Cells (MDSC)

Myeloid derived suppressor cells (MDSC) represent a heterogeneous, immature population of myeloid (precursor) cells that are commonly characterised by the expression of the myeloid lineage markers Gr1 and CD11 b in mice and are known to inhibit T effector responses. Myeloid cells with anti-inflammatory functions have been recognized in tumor-bearing mice for a long time (Young et al., 1987), but their functional significance for tumor immune evasion, angiogenesis and metastasis has only recently been more broadly appreciated (Nagaraj et al., 2007; Yang et al., 2008). MDSC have also been implicated in other pathological conditions like autoimmunity. Expansion of MDSC with T cell suppressive properties during sepsis in mice has been reported (Delano et al., 2007). Unlike for many cancers, their contribution to disease progression and host survival during infections is still undefined.

Interestingly, MDSC have also been shown to play a protective role in models of autoinflammatory diseases like IBD (Haile et al., 2008). Importantly their presence has also been demonstrated in humans (Hoechst et al., 2008).

As mentioned above, MDSC have pro-tumorigenic functions and inhibition of MDSC is considered a promising future therapeutic strategy (Gabrilovich and Nagaraj, 2009). In humans MDSCs have been characterized by various surface markers including CD34, CD33, CD15, and CD13, CD14-/lin-, or CD11b, CD66, VEGFR.

There is an unmet need for therapeutic agents to treat dysregulated inflammatory diseases or conditions, in particular sepsis and SIRS. In addition, immune evasion by cancers represents a major problem, which could be targeted through the same mechanisms that control inflammation during sepsis and SIRS.

### SUMMARY OF THE PRESENT INVENTION

Using a liver specific gp130-knock out mouse, it is demonstrated that hepatocyte derived SAA and CXCL1/KC are crucial regulators of innate inflammation during polymicrobial sepsis in mice. Employing complementary loss and gain of function experiments clear evidence for the protective role of SAA during sepsis, by controlling inflammation and promoting survival, without affecting bacterial clearance as provided. Thus a major mechanism of protection mediated by liver derived SAA and KC as described in greater detail in the relevant section below has been delineated.

Further, it is discovered that the chemokine CXCL1/GRO1 (KC in mice) is strongly upregulated in hepatocytes in a gp130-dependent manner during polymicrobial sepsis in mice, which correlates directly with its serum levels.

The present invention provides a novel therapeutic strategy for the treatment of dysregulated inflammation, in particular acute uncontrolled inflammation during sepsis, severe sepsis, septic shock and SIRS. The current invention proposes the use of recombinant proteins or their biologically active motifs in effective amounts of SAA or, as a reference, CXCL1/GRO1 to control dysregulated inflammation and prevent subsequent organ damage or death. Administration of these compounds actively promotes the function of MDSC.

That is, the present invention provides a pharmaceutical composition containing an active ingredient, wherein the active ingredient is an effective amount of SAA alone or active fragment thereof, or a pharmaceutically acceptable salt thereof for the use in the treatment of dysregulated inflammatory diseases or conditions by influencing the activity of myeloid-derived suppressor cells (MDSC), wherein the dysregulated inflammatory diseases or conditions are autoinflammatory diseases or autoinflammatory diseases with autoimmune components or autoimmune diseases including IBD, rheumatoid arthritis, psoriasis, multiple sclerosis, transplant rejection, graft vs. host and host vs. graft disease local and systemic inflammatory diseases, a systemic inflammatory response syndrome, sepsis, severe sepsis, septic shock, multi organ dysfunction syndrome, multiple organ failure syndrome and acute transplant rejection.

Preferably, said dysregulated inflammatory diseases or conditions are SIRS, sepsis, severe sepsis, septic shock, multi organ dysfunction syndrome (MODS), MOFS, or acute transplant rejection.

In another embodiment, the present invention provides a pharmaceutical composition containing an active ingredient, wherein the active ingredient is an effective amount of SAA alone or active fragment thereof, or a pharmaceutically acceptable salt thereof for use in the reduction, amelioration or termination of inflammatory responses or slow down the propagation or further escalation of inflammation or a disease associated with dysregulated inflammatory diseases or conditions by enhancing the activity of MDSC.

In another embodiment, the present invention provides a pharmaceutical composition, wherein the active ingredient is an invariant peptide at position 33-43 of SAA.

It is also preferred that the pharmaceutical composition further comprises pharmaceutical additives or auxiliary substances and, preferably, a pharmaceutically acceptable carrier, excipient or diluent.

In a second aspect, the present invention provides an effective amount of SAA alone or active fragments thereof or pharmaceutically acceptable salts thereof for use in treatment of sepsis.

Finally compounds as described herein for use in dysregulated inflammatory diseases or conditions are contemplated.

### BRIEF DESCRIPTION OF THE FIGURES

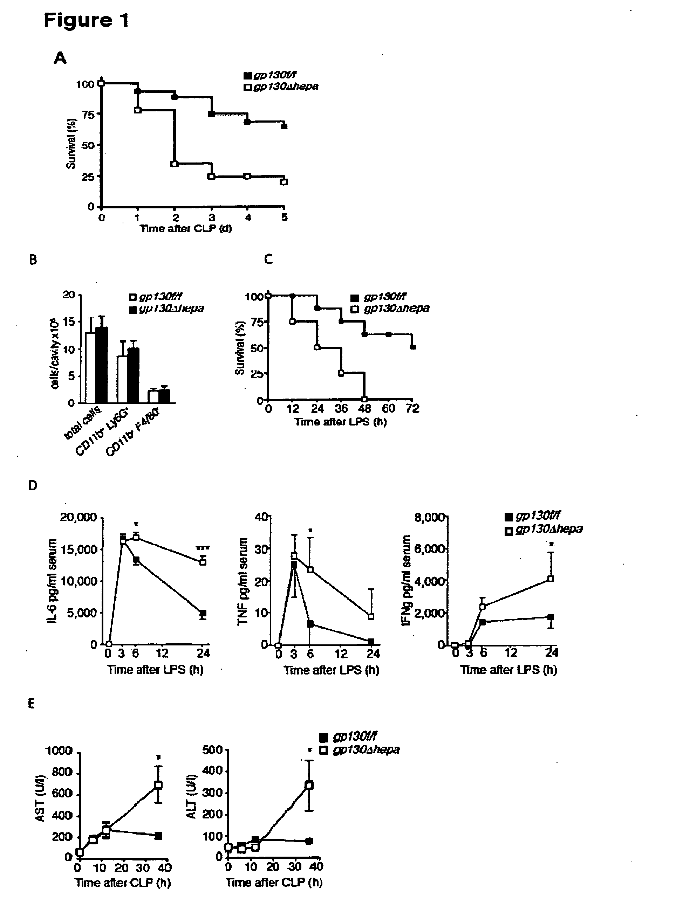

*Fig. 1**. Gp130*/*Stat3 signaling in hepatocytes is protective during polymicrobial sepsis and tempers the inflammatory response.*
   (**A**) Decreased survival of *gp130*Δ*hepa* mice after CLP (n= 45 per group, pooled data of 6 independent experiments). (**B**) Recruitment of immune cells to the peritoneal cavity was equivalent in *gp130Δhepa* and control mice. Peritoneal cavities were lavaged 18h after CLP, and infiltrating cells were counted and analyzed by flow cytometry. CD11b⁺Ly6G⁺; neutrophils, CD11b⁺F4/80⁻, monocytes and macrophages (n=5 per group, 1 of 3 independent experiments shown). (C) Decreased survival of *gp130*Δ*hepa* mice during endotoxic shock. Mice were treated with 20mg/kg LPS and survival was determined. (n= 10 per group, pooled data of 3 independent experiments). (**D**) Serum cytokine levels in *gp130f*/*f* and *gp130Δ hepa* mice after LPS treatment were measured by ELISA (8 per group, pooled data of 3 independent experiments). **(E)** Increased liver damage in *gp130Δhepa* mice after CLP. Serum levels of AST (left) and ALT (right), (n= 5 per group, 1 out of 3 independent experiments shown) (**J**) Representative H&E staining of histological sections of liver tissue. (*; p< 0.05, **; p<0.01, ***; p<0.001, data are presented as mean ±SEM)

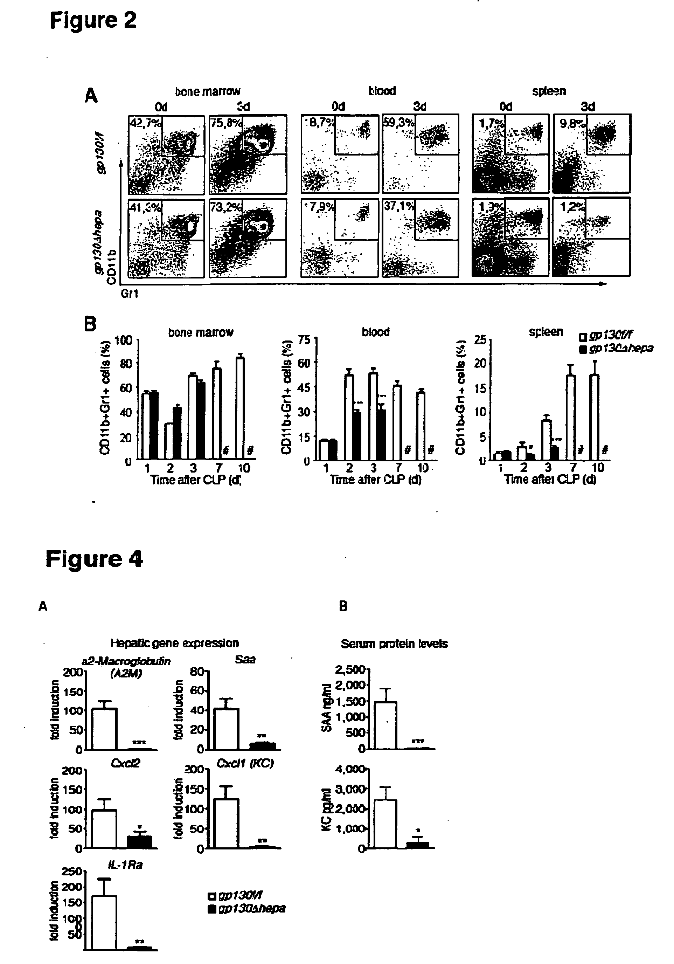

*Fig. 2**. Mobilization and splenic accumulation of CD11b*+*Gr1*+ *MDSC is impaired in gp130Δhepa mice.*
   (**A**) Representative flow cytometric analysis and (**B**) graphic analysis of CD11b⁺Gr1⁺ cell frequencies in bone marrow, peripheral blood and spleens in gp130f/f and *gp130Δhepa* mice at indicated time points after CLP (n= 5-12 per time point per group, pooled data of 12 independent experiments. #; *gp130f*/*f* did not survive to 7 and 10 d in sufficient numbers and could therefore not be included in the analysis). (*; p< 0.05, **; p<0.01, ***; p<0.001)

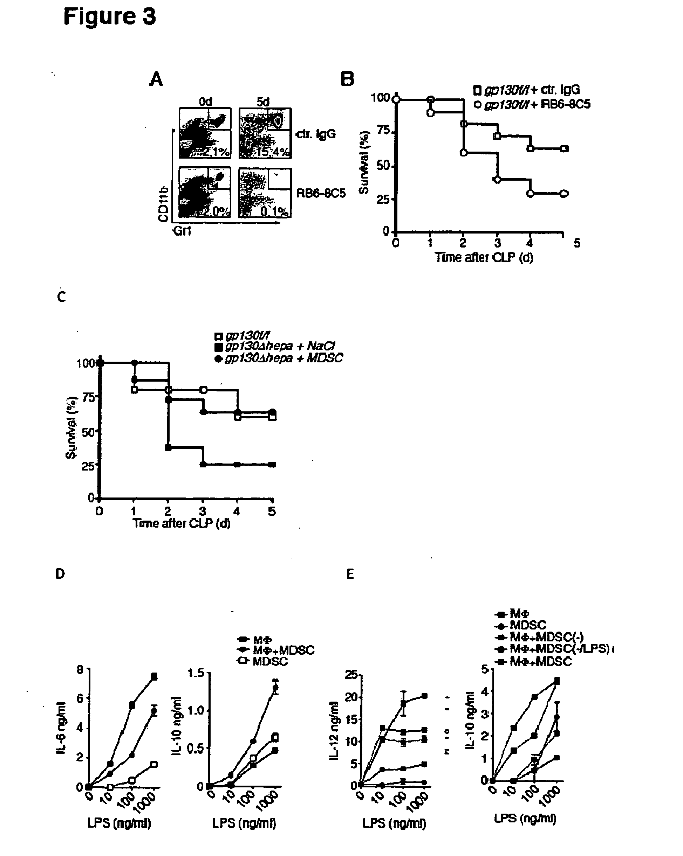

*Fig. 3**. MDSC regulate inflammatory responses and confer protection during sepsis.*
   (**A,B**) Depletion of Gr1 expressing cells decreased survival after CLP. *Gp130f*/*f* mice were injected with monoclonal antibodies against Gr1 (RB6-8C5) 24 h before and after CLP. Efficient depletion of MDSC was determined by flow cytometry of splenocytes (A). Survival was monitored (B, n= 9 per group, pooled data of 3 independent experiments). (**C**) *Gp130Δhepa* mice received either 5x10⁶ MDSC or saline 1 h and 24 h after CLP and survival was determined (n= 12 per group, pooled data of 3 independent experiments). (**D**) MDSC skew LPS induced cytokine production of peritoneal macrophages (MΦ). Macrophages were isolated from untreated *gp130f*/*f* mice and co-cultured with MDSC at a ratio of 1:3 and stimulated with LPS at indicated concentrations. Cytokine release was determined by ELISA (1 of 5 independent experiments shown). (**E**) Gr1⁺CD11b⁺ were isolated from spleens of septic mice (MDSC) or naïve mice (MDSC(-)) and co-cultured with macrophages. Some cells from naïve mice were pre-treated with 100ng/ml LPS in vitro prior to co-culture with macrophages (MDSC(-/LPS)). ( **E**: 1 of 3 independent experiments shown). (*; p< 0.05, **; p<0.01, ***; p<0.001, data are presented as mean ±SEM)
*Fig. 4**. Hepatocytes launch a specific gp130-dependent transcriptional program in response to infection.*
   (**A**) Quantitative real time PCR of liver samples taken from untreated *gp130f*/*f* and *gp130Δhepa* mice and 12 h after induction of sepsis (n= 8 per group, pooled data of 3 independent experiments). Acute phase proteins (alpha-2-macroglobulin, SAA), chemokines (KC, CxCI-2) and anti-inflammatory cytokines (IL-1Ra) were analyzed. (**B**) Serum protein levels of SAA (upper graph) and KC (lower graph) were analyzed by ELISA in *gp130f*/*f* and *gp130Δhepa* mice 24 h after CLP (n= 7 per group, 3 independent experiments). (*; p< 0.05, **; p<0.01, ***; p<0.001, data are presented as mean ±SEM)

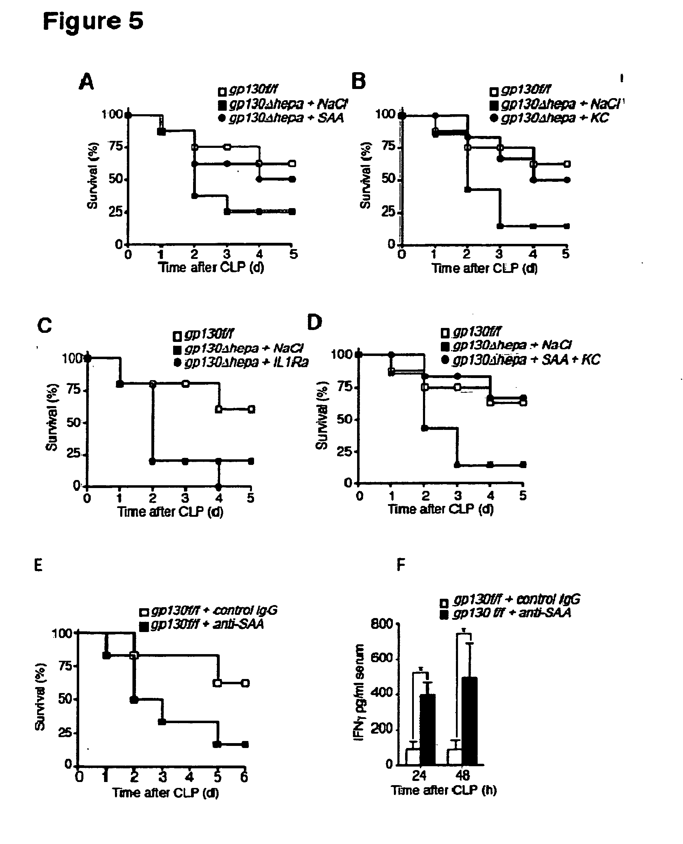

*Fig. 5**. Recombinant SAA and KC are protective during sepsis.* Survival of *gp130f*/*f* mice compared to *gp130Δhepa* mice treated with (**A**) SAA, (**B**) KC, (**C**) IL-1Ra (Anakinra) or (**D**) SAA and KC in combination or equal volumes of NaCl 1 h and 24 h after CLP (n= 9 per group, pooled data of 3 independent experiments). (**E**) Survival of *gp130f*/*f* mice treated with neutralizing anti-SAA antibodies or inactive control isotype IgG. (**F**) IFN-γ serum levels at indicated time points after CLP in *gp130f*/*f* mice treated with anti-SAA or control antibodies (n= 6 per group, pooled data of 2 independent experiments). (*; p< 0.05, data are presented as mean ±SEM).

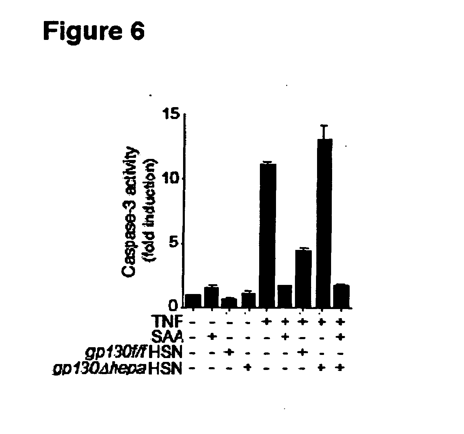

*Fig. 6**. Hepatocyte derived SAA reconstitute splenic MDSC and protects them from apoptosis.*
   (A) *In vitro* apoptosis of MDSC induced by TNF can be inhibited by SAA or supernatants from primary hepatocytes isolated from *gp130f*/*f* (*gp130f*/*f* HSN) but not from *gp130Δhepa* (*gp130Δhepa* HSN) mice. Hepatocytes were stimulated with the potent APP inducer and gp130 ligand leukemia inhibitory factor for 6 h. Apoptosis of MDSC was determined by measuring Caspase-3 activity. (1 of 3 independent experiments shown. Data are presented as mean ±SEM).

### DETAILED DESCRIPTION OF THE PRESENT INVENTION

The present inventors found that the hepatic APPs, like SAA, CRP and CXCL1, are critical to control inflammatory responses during dysregulated inflammatory diseases, sepsis, and administration of recombinant APPs represent an efficient treatment of dysregulated inflammatory diseases. They found that these protective effects are mediated at least in parts by promoting the activity of MDSC. The present inventors discovered a hitherto unrecognized role for MDSC in protecting the host against overshooting inflammation e.g. during sepsis, without affecting anti-microbial defence and bacterial clearance. Thus, SAA is useful for the treatment of dysregulated inflammatory diseases or conditions. SAA promotes MDSC mobilisation, accumulation and survival. Peripheral accumulation of MDSC inhibits excessive inflammatory responses. Therefore, targeting MDSC during sepsis through administration of SAA represents a novel promising therapeutic option.

In addition, regulating MDSC activity, in particular MDSC mobilisation, accumulation and survival allows treating of systemic inflammatory response syndrome, sepsis, severe sepsis, septic shock and MODS. The present inventors discovered that the gp130 depended communication between the liver and MDSC through acute phase proteins SAA, CRP and CXCL1 critically controls inflammatory responses or conditions, e.g. during infection.

Hence, in a first aspect, the present invention relates to a pharmaceutical composition containing an active ingredient, wherein the active ingredient is an effective amount of SAA alone or active fragment thereof, or a pharmaceutically acceptable salt thereof for the use in the treatment of dysregulated inflammatory diseases or conditions by influencing the activity of myeloid-derived suppressor cells (MDSC), wherein the dysregulated inflammatory diseases or conditions are autoinflammatory diseases or autoinflammatory diseases with autoimmune components or autoimmune diseases including IBD, rheumatoid arthritis, psoriasis, multiple sclerosis, transplant rejection, graft vs. host and host vs. graft disease, local and systemic inflammatory diseases, a systemic inflammatory response syndrome, sepsis, severe sepsis, septic shock, multi organ dysfunction syndrome, multiple organ failure syndrome and acute transplant rejection

In a preferred embodiment, the pharmaceutical composition contains as an active ingredient an effective amount of SAA alone or active fragments thereof, or a pharmaceutically acceptable salt thereof for use in the reduction, amelioration or termination of inflammatory responses or slow down the propagation or further escalation of inflammation or a disease associated with dysregulated inflammatory diseases or conditions by enhancing the activity of MDSC.

Moreover, according to another aspect, a pharmaceutical composition is provided for use in the treatment of autoinflammatory diseases or autoinflammatory diseases with autoimmune components or autoimmune diseases including IBD, rheumatoid arthritis, psoriasis, multiple sclerosis, transplant rejection, graft vs. host and host vs. graft disease, local and systemic inflammatory diseases.

In a preferred embodiment, the active ingredient is an effective amount of SAA alone or active fragment thereof, or a pharmaceutically acceptable salt thereof for use in the reduction, amelioration or termination of inflammatory responses or slow down the propagation or further escalation of inflammation or a disease associated with dysregulated inflammatory diseases or conditions by enhancing the activity of MDSC.

It is further preferred, that the active ingredient is an invariant peptide at position 33-43 of SAA.

In another preferred embodiment of the invention, the pharmaceutical composition further comprises pharmaceutical additives or auxiliary substances and, preferably, a pharmaceutically acceptable carrier, excipient or diluent.

In a second aspect of the present invention, an effective amount of SAA alone or active fragments thereof or pharmaceutically acceptable salts thereof is provided for use in treatment of sepsis.

According to the present invention, the term "serum amyloid A" (SAA) refers to the full length SAA molecules known as SAA1, SAA2, etc. The term also contemplates analogs or functional fragments of SAA, in particular of human SAA of gene bank accession No. NM_000331 (Seq. ID No. 5), and NM_030754 (Seq. ID. No. 7), P02735.

As used herein, the term "C reactive protein" (CRP) refers to the full length protein, e.g. the human protein NM_000567 (Seq. ID. No. 9), P02741. The present invention contemplates analogs as well as functional fragments of C reactive protein.

The term "CXCL1" as used herein refers to the CXCL1 chemokine also known as KC or GR01, having the gene bank acsession number J03561 and NM 001511(human form), Seq. ID. No. 3, P09341 . The present invention contemplates analogs as well as functional fragments of CXCL1.

As used herein, the term "a functional fragment thereof" refers to a fragment of the respective protein, e.g. a peptide which display the same activity. In the present document, the term "SAA", "CRP" and "CXCL1" includes respective fragments thereof unless otherwise indicated. Further unless explicitly noted, said terms includes salts or solvates of the compounds.

The term "analog" as used herein refers to molecules having the same activity as SAA, CRP and CXCL1, respectively. In particular, an analog has the same activity as the respective full length proteins in activating and mobilising MDSC. The term "CRP", "SAA" and "CXCL1" includes respective analogs which may be present in a salt or solvate form unless otherwise indicated.

In a preferred embodiment, the SAA, CRP and CXCL1 molecules are proteins of SEQ ID Nos. 4, 6, 8, 10, respectively.

The active ingredients described herein may be in the form of pharmaceutically acceptable non-toxic salts thereof or other galenic preparations. Salts include acid added salts, such as salts with inorganic acids or with organic acids and also salts formed with cationic ions, like metallic ions, in particular alkali or alkaline health metal 1 or NH4+. Further, the active ingredients may be present in form of solvates thereof (e.g. hydrates).

In addition, the pharmaceutical composition comprising as an active ingredient SAA alone is useful in the therapy of systemic inflammatory response syndrome, sepsis, severe sepsis, septic shock, multi organ dysfunction syndrome, or acute transplant syndrome.

Moreover, it is preferred that the pharmaceutical composition comprises as an active ingredient an effective amount of SAA alone or a pharmaceutically acceptable salt thereof.

In preferred embodiments, the pharmaceutical composition further comprises pharmaceutical additives or auxiliary substances, preferably, a pharmaceutically acceptable carrier, excipient or diluent.

The term "pharmaceutical composition" means a composition suitable for pharmaceutical use in a subject or individual, including an animal or human. A pharmaceutical composition generally comprises an effective amount of an active agent or ingredient and a carrier, including e.g. a pharmaceutically acceptable carrier.

A "therapeutic treatment" is a treatment administered to a subject or an individual to display symptoms or signs of pathology, disease, or disorder in which treatment is administered into the subject for the purpose of diminishing or eliminating those signs or symptoms of pathology, disease or disorder.

As used herein, the term "carrier" refers to a diluent, adjuvant, excipient or vehicle.

The composition comprising the compounds according to the present invention will be formulated, dosed, and administered in a fashion consistent with good medical practice. Factors for consideration in this context include the stage of the particular disease or disorder being treated, the particular mammal being treated, the clinical condition of the individual subject, the site of delivery of the agent, the method of administration, the scheduling of administration, and other factors known to medical practitioners. The effective amount of the agent to be administered will be governed by such considerations. The key factor in selecting an appropriate dose and scheduling is the result obtained, as indicated above. The compounds according to the present invention are administered by any suitable means, including parenteral, topical, subcutaneous, intraperitoneal, intrapulmonary, intranasal, and/or intralesional administration. Parenteral infusions include intramuscular, intravenous, intraarterial, intraperitoneal, or subcutaneous administration. Intrathecal administration is also contemplated.

"Therapeutically- or pharmaceutically-effective amount" as applied to the compositions of the instant invention refers to the amount of composition sufficient to induce a desired biological result. That result can be alleviation of the signs and markers, symptoms, or causes of a disease, or any other desired alteration of a biological system. In the present invention, the result will typically influence inflammatory response, including cytokine inflammatory mediator levels as well as frequency and activity of MDSC either by activating or mobilising said cells or by suppressing their activity including apoptosis induction.

In vitro assays as well as animal models may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" means administration of a therapeutically effective dose of the aforementioned pharmaceutical composition comprising the active ingredient according to the present invention in form of salts and solvates thereof to an individual.

The pharmaceutical composition according to the present invention comprises the active ingredient as described herein and, optionally, a pharmaceutical acceptable carrier. Such pharmaceutical compositions comprise a therapeutically effective amount of the active ingredient as defined herein and, optionally, a pharmaceutically acceptable carrier. The pharmaceutical composition may be administered with a physiologically acceptable carrier to a patient, as described herein. Acceptable means that the carrier be acceptable in the sense of being compatible with the other ingredients of the composition and not be deleterious to the recipient thereof. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, aerosols and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds, salts or solvates thereof, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in a unit dosage form, for example, as a dry lyophilised powder or water free concentrate in a hermetically sealed container such as an ampoule or sachette indicating the quantity of active agent. Where the composition Is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use according to the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

These and other embodiments are disclosed and encompassed by the description and examples of the present invention. Further literature concerning any one of the methods, uses and compounds to be employed in accordance with the present invention may be retrieved from public libraries, using for example electronic devices.

In a further embodiment, the present invention relates to the use of an effective amount of SAA alone or pharmaceutically acceptable salts thereof for activation and mobilisation as well as for survival of MDSC e.g. for treating excessive dysregulated inflammatory diseases.

That is, when excessive dysregulated inflammatory diseases, like it is the case in sepsis, occurs in an individual, the compounds according to the present invention are administered to said individual afflicted with an excessive dysregulated inflammatory disease to ameliorate to slow down said inflammation, or to reduce escalation of the inflammation or to terminate inflammatory responses.

Further, an effective amount of, SAA alone or pharmaceutically acceptable salts or solvates thereof may be used for suppressing inflammation in individuals afflicted with dyregulated inflammatory diseases or conditions.

The following examples are intended to illustrate the invention further.

### EXAMPLES

### MATERIAL AND METHODS

Mice. Male *gp130f*/*f, gp130Δhepa, gp130StatΔhepa* and *gp130RasΔhepa* mice at the age of 8-12 weeks, as described previously (Klein et al., 2005) have been used. All experiments were approved by the institutional Animal Care and Use Committee. All experiments were carried out in agreement with the 'Guide for the Care and Use of Laboratory Animals' (NIH publication 86-23, revised 1985).

**Cecal ligation and puncture (CLP).** Polymicrobial sepsis was induced by CLP. Briefly, mice were anesthetized; the cecum was ligated and punctured twice with a 20G needle. Mice were monitored every 6 h for 24 h then every 12 h till the end of the experiment (between 5 and 10 days).

**LPS-induced endotoxic shock.** Mice received 20mg/kg LPS intraperitoneally and were monitored every 6 hours for 24 h then every 12 h till the end of the experiment (between 3 and 5 days).

**Cell isolation and culture.** MDSC were isolated from spleens of *gp130f*/*f* mice 5 days after CLP. After sacrifice, spleens were explanted and splenocytes were isolated by collagenase digestion. Red blood cells were lysed using Pharmlyse lysing buffer (BD Biosciences). MDSC were purified from cell suspension by magnetic cell sorting (MACS, Miltenyi Biotec) using anti-CD11b conjugated magnetic beads (Miltenyi Biotec). Purity was determined by flow cytometry. Only cell preparations with a purity of >90% for further experiments were used. Alternatively, mice were injected with 5mg/kg LPS i.p. and 5 days later CD11b⁺Gr1⁺ splenocytes were purified by FACS sorting (Influx cell sorter, BD Biosciences). For overnight culture experiments cells were kept in RPMI 1640 (Invitrogen) supplemented with 10% heat inactivated fetal calf serum (Sigma). MDSC were cultured with the following substances as indicated, murine TNF alpha (Peprotech), LPS (Sigma), SAA (Peprotech).

For generation of peritoneal macrophages mice were injected with of 1ml thioglycollate (BD Biosciences) intraperitoneally. Total cells were harvested after 72 h and plated at 1x10⁶/ml in RPMI 1640 (Invitrogen) supplemented with 10% fetal calf serum (Sigma) and 1% penicillin/streptomycin (Invitrogen). After overnight culture non-adherent cells were removed and purity of macrophages was confirmed by F4/80 staining and flow cytometry.

Primary hepatocytes were isolated from *gp130f*/*f* and *gp130Δhepa* mice by collagenase perfusion as described previously (Klein et al., 2005), 1x10⁶ cells were cultured on 6-cm culture plates with DMEM (Invitrogen) containing 4.5 g/L glucose, 10% FCS, and penicillin/streptomycin overnight. Cells were stimulated with 50 ng/ml leukemia inhibitory factor LIF (Peprotech) for 6 h, and supernatants were harvested and stored at -80°C.

**In vitro culture of MDSC.** Culture conditions have been used previously (Delano et al., 2007). After MACS purification of splenic MDSC from control *gp130f*/*f* mice five days after CLP. Cells were plated at 10⁶ cells/ml in RPMI 1640 supplemented with 10% fetal calf serum, 2 mM I-glutamine, 200 U/ml penicillin, and 50µg/ml streptomycin, and cultured overnight with the indicated compounds. For in vitro suppression assays, purified MDSC were co-cultured with peritoneal or bone marrow derived macrophages, stimulated with LPS (Sigma) and subsequent cytokine release was measured by ELISA. In some cases MDSC were separated from macrophages in transwells (pore size 0.4 µm, Corning Inc) For in vitro migration studies, splenic or bone marrow derived CD11b⁺Gr1⁺ cells were seeded in transwells (pore size 3µm, Corning Inc.) and increasing amounts of recombinant KC (Peprotech) were added to the lower well. After 3h migrated cells were counted in a hemocytometer and the migration index of migrated/non-migrated cells was calculated.

**Adoptive MDSC transfer.** MDSC were isolated as described above, *gp130Δhepa* mice received 5x10⁶ MDSC intravenously 1 h and 24 h after CLP. Control mice were treated with equal volumes of NaCl.

**Depletion of Gr1⁺ cells.** Monoclonal antibodies against Gr1 from RB6-8C5 hybridoma cells (Tepper, R.I., et al, 1992, Science 257, 548-551) have been used and *gp130f*/*f* mice received 10 mg/kg of antibodies 24 h before and 24 h after CLP. Efficiency of depletion was monitored by FACS. Control mice received non-specific isotype IgG antibodies.

**Treatment of animals with recombinant SAA, KC and IL1Ra.** Mice received 0.8 mg/kg recombinant SAA (Peprotech) or 40µg/kg KC (Peprotech) or a combination of both intravenously 1 h and 24 h after CLP. Control mice received the same volume of saline. IL-1Ra (Anakinra) (Kineret, Amgen) was injected subcutaneously every 4 h over a period of 24 h.

**Systemic neutralization of SAA.** Mice received 50mg/kg of a combination of anti-SAA monoclonal antibodies (**mc29** and **mc4**, 1:1) directed against the highly conserved (invariant) region of SAA (see above) i.p. 12h before and 33mg/kg 12h after CLP. Control mice received equivalent amounts of inactive control antibodies (mc1). The three antibodies are described in Linke et.al., J. Histochem, Cytochem. 32. 322328 (1984).

**Cytokine measurements.** Serum samples were collected by bleeding at the indicated time points. IL-6, TNF, IFN-γ and KC were measured by ELISA (R&D systems). SAA was measured by ELISA (Biosource). IL-6, TNF, IL-12 and IL-10 were measured from cell culture supernatants by ELISA (R&D systems).

**Flow cytometry.** Cells suspensions were incubated with the flourochrome conjugated antibodies listed below and analyzed with a BD FACS Canto II (BD Biosystems). CD45, Gr1 (both BD Biosciences), CD11b (eBioscience).

Gene-microarray analysis and real time PCR. *Gp130f*/*f* and *gp130Δhepa* mice were sacrificed without prior treatment or 12 h after CLP. Livers were explanted and tissue samples were frozen immediately. Liver RNA samples from 3 mice per experimental group were used for microarray analysis. RNA samples were prepared and hybridized on Affymetrix GeneChip Mouse Genome 430 2.0 arrays. Gene expression levels were calculated applying the GC content-corrected robust multichip average algorithm (GCRMA) and a remapped Gene Chip Description File (CDF). Differentially expressed genes were identified using Limma statistics. Genes with a corrected p-value similar or below 0.05 and/or fold change above 1.5 or below -1.5 were considered significantly regulated. Detailed descriptions of the applied methods are available on request. Ingenuity Pathway Analysis (v5.5, Ingenuity Systems) and ErmineJ Gene Score Resampling were used to identify the most significantly changed canonical signaling pathways or Gene Ontology (GO) biological processes in *gp130f*/*f* and *gp130Δhepa* mice 12 h after sepsis onset, using a p-value similar to or below 0.05 as input criteria. Fold changes of selected genes were visualized in a heat map diagram.

Real time PCR analysis was performed in order to confirm data from the gene array. RNA was isolated using RNeasy columns (Qiagen) following the manufacturer's instructions. First strand synthesis was performed with Oligo dT primers and reverse transcription with M-MLV Reverse Transcriptase (Invitrogen). Quantitative Real Time PCR was performed using SYBR Green Reagent (Invitrogen Corp.) in ABI-Prism 7300 Real Time PCR system (Applied Bioscience). Reactions were performed in duplicate and GAPDH values were used to normalize gene expression.

**Determination of bacterial burden.** Mice were sacrificed 24 h after CLP. The peritoneal cavity was lavaged and the lavage fluid was plated on blood-agar plates and McKonkey-agar plates in serial dilutions. Spleens were removed, homogenized and plated in serial dilutions on agar plates. Colony forming units were counted after overnight incubation.

**Determination of apoptosis by Caspase-3 kinase activity.** Purified MDSC were cultured overnight with the indicated compounds. Then cells were washed and lysed at 4°C in 5 mmol/L Tris/HCl, pH 8.0, 20 µmol ethylenediaminetetraacetic acid, 0.5% Triton X-100. Lysates were clarified by centrifugation at 13,000*g* for 10 min. Reaction mixture contained 30 µg cellular lysates,1000 µl assay buffer (20 mmol/L HEPES, pH 7.5, 10% glycerol, 2 mM dithiothreitol), and 20 mM caspase-3 substrate (Ac-DEVD-AMC) (BD Pharmingen). After 2 h incubation in the dark, enzymatic activity was measured in a Luminiscence Spectophotometer (LS-50; Perkin Elmer).

**Determination of apoptosis by annexin-V staining.** Cells were stained for CD45, Gr1 and CD11b, followed by incubation with annexin-V - FITC (BD Biosciences) and analyzed by flow cytometry.

**Statistical analysis.** Statistical significance was determined by unpaired two-tailed student's t-test implemented in Excel (version 11.0, Microsoft)

Herein it is shown that hepatic gp130-deficiency severely decreased survival of *gp130Δhepa* mice during polymicrobial sepsis compared to control littermates (Fig. 1A). Control mice carried loxP sites flanking Exon 16 of the gp130 gene, but did not express Cre-recombinase (*gp130f*/*f*)*.* The protective effects of hepatic gp130 signaling required Stat3 activation, demonstrated by a markedly decreased survival of *gp130StatΔhepa* compared to *gp130f*/*f* and *gp130RasΔhepa* animals. Proinflammatory cytokines like IL-6, TNF and interferon gamma (IFN-γ) are heavily involved in the pathogenesis of septic shock and subsequent organ failure. In the mouse model described herein, serum levels of these cytokines peaked at 6 h in all animals. In contrast, while the systemic cytokine levels quickly normalized in *gp130f*/*f* control and *gp130RasΔhepa* mice, they remained persistently elevated after 24 and 48 h in *gp130Δhepa* and *gp130StatΔhepa* animals.

Then, the possibility of defective bacterial clearance in mice lacking hepatic gp130/Stat3 signaling leading to elevated cytokine levels was explored. However, when examining bacterial counts of the peritoneal cavity and the spleen, by plating serial dilutions of peritoneal lavage fluid or spleen homogenates 12h after CLP, no differences between the groups have been found and nearly all animals had cleared the bacteria from the spleens after 36h. Furthermore, leukocyte recruitment to the peritoneal cavity was equal in all groups (Fig. 1B) indicating that the innate immune response was intact. This suggested that impaired counter regulation of the initial inflammatory response rather than defective anti-bacterial defense was responsible for the exacerbated inflammation and increased mortality of *gp130Δhepa* mice. To further test this hypothesis sterile endotoxic shock by injection of lipopolysaccharide (LPS) was induced. Similar to the infection model, hepatic gp130-deficiency strongly increased mortality (Fig. 1C) and caused prolonged inflammation (Fig. 1D). Further liver-intrinsic effects of gp130-deficiency during sepsis has been studied and it has been found that *gp130*Δ*hepa* mice showed markedly elevated liver enzyme serum levels (Fig. 1E) and pronounced hepatocyte necrosis compared to *gp130f*/*f* mice following CLP. Enhanced liver injury not only reflected the severity of the systemic inflammation but also confirmed the auto-protective function of IL-6/gp130 signaling in hepatocytes. Collectively, these experiments suggested that liver derived factors were essential to control the inflammatory response to infection.

Next it was investigated whether dysregulated inflammation in *gp130Δhepa* mice was associated with changes in the composition of immune cells in the peripheral blood, lymphoid organs and the liver. Especially it was focused on MDSCs, an immature population of myeloid (precursor) cells characterized by surface expression of CD11b and Gr1 in mice. MDSCs have recently gained significant attention for their role in malignancies. Interestingly, one previous study reported expansion of MDSCs in the bone marrow and accumulation in the spleen of septic mice (Delano et al., 2007). Therefore the expansion of CD11b⁺Gr1⁺ cells in the bone marrow, as well as their mobilization to the peripheral blood and accumulation in the spleen, lymph nodes and the liver of mice at various time points after induction of polymicrobial sepsis have been monitored. As shown herein although CD11b⁺Gr1⁺ cells expanded similarly in the bone marrow of all mice, the population of circulating CD11b⁺Gr1⁺ cells in the blood was significantly reduced in *gp130Δhepa* mice (Fig. 2A, B). Further accumulation of MDSCs in the spleens of septic *gp130f*/*f* control mice as previously reported (Delano et al., 2007) (Fig. 2A, B) has been observed. However, splenic enrichment of MDSCs after CLP was completely blocked in *gp130Δhepa* animals, as demonstrated by flow cytometry and immunofluorescence microscopy (Fig. 2A, B). Hepatic accumulation of CD11b⁺Gr1⁺ cells was also significantly decreased in *gp130Δhepa* mice. No increase of MDSCs in mesenteric lymph nodes was observed in any of the animals. These data strongly suggested that gp130-dependent signals in the liver facilitated mobilization and peripheral accumulation of MDSCs during polymicrobial sepsis.

Positive CD11b/Gr1 staining is not unique to MDSCs especially in compartments with relatively high numbers of myeloid cells like the bone marrow and the peripheral blood. The population of CD11b⁺Gr1⁺ cells is therefore not necessarily equivalent to MDSCs. In the absence of exclusive surface markers MDSCs are best defined functionally, through their ability to suppress immune responses. Therefore, their functions during sepsis were analyzed. Myeloid cells with anti-inflammatory functions have been recognized in tumor-bearing mice for a long time (Young et al., 1987), but their functional significance for tumor immune evasion, angiogenesis and metastasis has only recently been more broadly appreciated (Nagaraj et al., 2007; Yang et al., 2008). MDSCs have also been implicated in other pathological conditions like autoimmunity (Haile et al., 2008; Zhu et al., 2007). However, despite significant advances in cancer, there is only limited data on MDSC functions in infections and sepsis. Expansion of MDSCs with T cell suppressive properties during sepsis in mice has been reported (Delano et al., 2007). Unlike for many cancers, their contribution to disease progression and host survival during infections is still undefined. Thus, a goal of the present invention is to establish a link between hepatic gp130 deficiency, subsequent impairment of MDSC accumulation and reduced survival. Two independent approaches to test the relevance of MDSCs for host survival have been chosen. First Gr1⁺ MDSCs were depleted in control mice using a monoclonal antibody directed against Gr1. Depletion of MDSCs significantly increased mortality compared to mice treated with a control isotype antibody (Fig. 3A,B). Since anti-Gr1 treatment is not specific for MDSCs and would also target other Gr1⁺ cells like neutrophils, it was decided to adoptively transfer MDSCs, isolated from spleens of *gp130f*/*f* donors 5 days after CLP, into *gp130Δhepa* animals. Purified MDSCs from spleens of *gp130f*/*f* mice showed a characteristic morphological heterogeneity and immature phenotype. Around 50% of CD11b⁺Gr1⁺ cells expressed Ly6G, a marker associated with the PMN subset of MDSCs. Importantly, MDSCs efficiently reversed the detrimental effects of hepatic gp130-deficiency during infection (Fig. 3C). MDSC transfer increased survival of *gp130Δhepa* mice to wildtype levels (Fig. 3C), demonstrating a key role of MDSCs in regulating inflammatory responses to infection. Previous studies had shown suppressive functions of MDSCs on T cells (Delano et al., 2007). Herein it is shown that purified sepsis-induced splenic MDSCs inhibited LPS-induced pro-inflammatory cytokine release while strongly inducing the production of IL-10 in primary macrophages (Fig. 3D, E). This skewing effect was dependent on activation of the MDSCs *in vivo.* CD11b⁺Gr1⁺ cells that were sorted from the spleens of untreated mice were strongly impaired in their ability to inhibit IL-12 release and induce IL-10 production in LPS treated macrophages (Fig. 3E). Pre-activation of CD11b⁺Gr1⁺ cells from naïve mice with LPS *in vitro* prior to co-culture with macrophages, partly restored IL-10 induction but failed to recover inhibition of IL-12 production (Fig. 3E). Thus, MDSCs generated during infection show a strong anti-inflammatory phenotype, in contrast to splenic CD11b⁺Gr1⁺ cells under steady state conditions. The anti-inflammatory functions of MDSCs were largely dependent on direct cell-cell contact. Separation of MDSCs and macrophages in a transwell system strongly reduced IL-10 induction, however it only partially blocked IL-12 suppression, indicating that additional soluble factors like TGF-β may play a role as well. The results shown herein suggest that direct interaction of MDSCs with innate effector cells may constitute a novel auto-regulatory mechanism of innate immune responses to infection.

Hitherto, knowledge about factors and conditions that induce MDSC production and function as well as their homing to peripheral tissues or lymphoid organs is incomplete. Observations made in murine cancer models have suggested that tumor derived factors induce the expansion of MDSCs and inhibit their differentiation into DCs by activation of Janus-Kinase (JAK)2/Stat3 pathways in MDSCs (Nefedova et al., 2004). In the present model, JAK2/Stat3 signaling in hematopoetic cells was intact and no differences in the expansion of CD11b⁺Gr1⁺ cells in the bone marrow (Fig. 2A, B) has been observed. Instead, hepatocyte specific deletion of gp130/Stat3 signaling abrogated the peripheral accumulation of MDSCs. Therefore, it was aimed to identify liver-derived gp130-dependent factors that were involved in mobilization and peripheral accumulation of MDSCs, thereby controlling inflammation. Transcriptional analysis of 16,475 genes by gene-microarray in liver tissue samples taken from untreated and septic *gp130f*/*f* and *gp130Δhepa* mice revealed a total of 3,936 genes that were regulated more than 1.5 fold 12h after CLP, 2,266 of which were differentially regulated by gp130. Focusing on secreted proteins, three groups of genes with a particularly strong gp130-dependent induction were noticed: 1. Classical APPs like alpha-2-macroglobulin, CRP or serum amyloid A (SAA), 2. chemokines with chemotactic activity on myeloid cells, like CXCL1 (KC), CXCL2 or Ccl3 (MIP-1α), and 3. anti-inflammatory cytokines like IL1-receptor antagonist (IL1Ra). These results were further confirmed by real time RT PCR (Fig. 4A). Accordingly, the serum levels of SAA and CXCL1 were strongly elevated in *gp130f*/*f* but not in *gp130Δhepa* mice 24 h after CLP, showing that hepatic gp130 signaling controls the systemic levels of these proteins (Fig. 4B). Transcript levels of IL-6 were also significantly reduced in *gp130Δhepa* mice compared to controls (8.3 vs. 1.9 fold induction, data not shown), which is noteworthy since it is widely believed that the liver is a major source of IL-6 and TNF during sepsis. In contrast it was found that *gp130Δhepa* mice showed elevated serum levels of IL-6 following CLP despite reduced hepatic IL-6 production. Hepatic TNF transcript levels were not found to be differentially regulated by gp130. This suggests a mainly extrahepatic production during sepsis, for example in macrophages activated by microbial products.

Having identified hepatic genes regulated by gp130 during polymicrobial infection, it was aimed to determine their functional contributions to the regulation of the inflammatory response and MDSC accumulation. To this end *gp130Δhepa* mice that failed to induce SAA, CXCL1 and IL1Ra have been treated with the respective recombinant proteins. Administration of SAA or CXCL1 alone partly restored survival (Fig. 5A,B), while a combination of both proteins completely rescued *gp130Δhepa* mice normalizing survival rates and systemic cytokine levels (Fig. 5A,B,D). In sharp contrast, treatment with the anti-inflammatory cytokine IL1Ra (Anakinra) resulted in 100% mortality of *gp130Δhepa* mice after 4d (Fig. 5C). Detrimental effects of IL-1Ra treatment may be due to impaired bacterial clearance or defective tissue repair in the absence of IL-1.

Notably, administration of recombinant KC, known as a potent chemoattractant for polymorphonuclear cells, significantly elevated the number of circulating CD11b⁺Gr1⁺ cells in septic *gp130*Δ*hepa* mice. Recombinant KC also induced chemotaxis of purified MDSCs in vitro. In contrast, splenic MDSC numbers were only marginally elevated in KC-treated *gp130Δhepa* animals. Thus, it was conceivable that KC was involved in mobilization of myeloid cells from the bone marrow, while accumulation of MDSCs in the spleen required additional factors. Therefore the ability of SAA to restore splenic MDSCs was tested. SAA is mostly regarded as a pro-inflammatory protein with activating properties on innate immune cells, and has also been implicated in the pathogenesis of chronic inflammation like atherosclerosis. SAA treatment of *gp130Δhepa* mice during sepsis efficiently reduced mortality (Fig. 5B,D) and inflammation but importantly, it nearly restored MDSC numbers in the spleen especially when given in combination with KC, demonstrating that SAA was sufficient to rescue *gp130Δhepa* mice during sepsis. However, it was not clear if SAA was required in the context of an intact APR. Blocking SAA activity in *gp130f*/*f* mice by injection of neutralizing anti-SAA antibodies strongly enhanced sepsis-related mortality and systemic cytokine levels (Fig. 5F,G). Given the large quantities and vast variety of APPs produced during sepsis, it was surprising to find that inhibition of SAA alone had such dramatic effects and it indicates a central role for this APP in the regulation of the inflammatory response to infection. Thus, hepatic APPs promote MDSC accumulation and regulate inflammation during sepsis. However, how MDSCs home to the spleen and not to other lymphoid organs like the mesenteric lymph node is not clear to this point and needs further investigation.

The liver might be a potent source of MDSC inducing proteins not only in sepsis but potentially also in cancer. In this respect it is also noteworthy that MDSCs have been shown to accumulate in human hepatocellular carcinoma, a cancer known to notoriously escape anti-tumor immune responses (Hoechst et al., 2008).

In addition to JAK2/Stat3, the role of which for MDSC induction has been studied mainly in malignancies, it was previously shown that the expansion of MDSCs during polymicrobial sepsis required MyD88 signaling (Delano et al., 2007). However, it is not clear if direct MyD88 activation in MDSCs, or alternatively indirect mechanisms like MyD88-dependent production of cytokines like IL-6 are responsible for these effects. The present invention does not directly address this issue, but the results demonstrate that MyD88/NFκB dependent IL-6 cytokines and subsequent gp130 signaling in hepatocytes is a crucial element for peripheral accumulation of MDSCs in sepsis. This concept is intriguing because it implicates a novel negative feedback mechanism of TLR-mediated inflammatory responses.

Contraction of immune cells, particularly lymphocytes, during severe infections is a well-known phenomenon. Here, it was found a higher percentage of AnnexinV⁺ apoptotic MDSCs in spleens of *gp130Δhepa* mice compared to *gp130f*/*f* mice. Interestingly, the apoptotic fraction was significantly reduced in SAA treated *130Δhepa* animals. Thus it was reasoned that SAA promotes survival of peripheral. Therefore the potential of recombinant SAA to inhibit apoptosis of MDSCs isolated from spleens of septic mice was tested. Indeed, it was found that both recombinant SAA and supernatant of stimulated primary hepatocytes from *gp130f*/*f* but not from *gp130Δhepa* mice prevented TNF-induced MDSC apoptosis (Fig. 6). Primary hepatocytes isolated from control *gp130f*/*f* and *gp130Δhepa* mice were stimulated with leukemia inhibitory factor, a strong gp130 agonist, to induce production of gp130 dependent proteins. Addition of SAA to the supernatant of *gp130Δhepa* hepatocytes fully restored the anti-apoptotic capacity (Fig. 6), demonstrating that liver derived SAA, produced in response to gp130 activation protects MDSCs from apoptosis. Therefore, SAA inhibits MDSC apoptosis in vitro and in vivo. The mechanism underlying the anti-apoptotic effect of SAA is not clear at present. Interestingly, SAA was recently identified as an endogenous TLR2 ligand activating MyD88 signaling (Cheng et al., 2008a). It is therefore conceivable that SAA activates TLR2/MyD88 in MDSCs to protect them from apoptosis. This would also be in line with the previously reported requirement for MyD88 for MDSC expansion during sepsis (Delano et al., 2007).

Collectively, these results define a pivotal role of liver derived proteins in regulating MDSC mobilization and survival in sepsis, and the production of these factors requires gp130/Stat3 signaling in hepatocytes.

In summary, the present invention demonstrates that MDSCs have potent host protective anti-inflammatory functions during polymicrobial infections. *Gp130Δhepa* animals that failed to accumulate MDSCs in the spleen suffered from dysregulated inflammation and increased mortality. Adoptive transfer of splenic MDSCs into *gp130*Δ*hepa* mice conferred protection. It is known that adaptive immune cells are required to control innate immune responses to infections (Kim et al., 2007). As shown here (Fig. 3) and previously in cancer-derived MDSCs (Sinha et al., 2007), MDSCs can directly interact with macrophages and inhibit proinflammatory cytokine release. Thus, they represent an auto-regulatory component of the innate immune system, contributing to carefully balance the inflammatory response to bacterial infection and sepsis.

The results as shown herein directly link MDSC functions during infection to hepatic APPs induced by gp130/Stat3 activation. Our study shows that APPs are crucial regulators of inflammatory responses to infection and highlights the close relationship between hepatocytes and innate immune cells and SAA plays a key role in this regulatory process.

Further embodiments that are not part of the invention are:
a pharmaceutical composition containing as an active ingredient an effective amount of i) SAA, CRP and/or CXCL-1 or analogs or active fragments thereof or ii) inhibitors of SAA, CRP and/or CXCL-1 activity, or a pharmaceutically acceptable salt of i) or ii) for the use in the treatment of dysregulated inflammatory diseases or coditions by influencing the activity of myeloid-derived suppressor cells (MDSC);
the pharmaceutical composition containing as an active ingredient an effective amount of SAA, CRP and/or CXCL-1 or analogs or active fragments thereof or a pharmaceutically acceptable salt thereof for use in the reduction, amelioration or termination of inflammatory responses or slow down the propagation or further escalation of inflammation or a disease associated with dysregulated inflammatory diseases or conditions by enhancing the activity of MDSC;
the pharmaceutical composition being used in the treatment of autoinflammatory diseases or autoinflammatory diseases with autoimmune components or autoimmune diseases including IBD, rheumatoid arthritis, psoriasis, multiple sclerosis, autoimmune hepatitis, transplant rejection, graft vs. host and host vs. graft disease, acute toxic and chronic toxic liver damage, local and systemic inflammatory diseases;
the pharmaceutical composition being in the therapy of a systemic inflammatory response syndrome, sepsis, severe sepsis, septic shock, multi organ dysfunction syndrome, multiple organ failure syndrome or acute transplant rejection;
the pharmaceutical composition containing as an active ingredient an effective amount of an inhibitor of SAA, CRP and/or CXCL-1 activity, in particular neutralising antibodies against SAA, CRP and/or CXCL-1, or a pharmaceutically acceptable salt thereof for use in the treatment of malignancies or dysregulated inflammatory disease or conditions whereby the inflammation response is suppressed, through inhibition of MDSC accumulation, frequency and/or activity;
the active ingredient being an effective amount of SAA, an analog or active fragment thereof, or an inhibitor of SAA, or a pharmaceutically acceptable salt thereof;
the active ingredient being an effective amount of SAA, an analog or an active fragment thereof, in combination with CXCL-1, an analog or active fragment thereof, or a pharmaceutically acceptable salt thereof;
the pharmaceutical composition comprising pharmaceutical additives or auxiliary substances and, preferably, a pharmaceutically acceptable carrier, excipient or diluent;
the use of an effective amount of SAA, CRP and/or CXCL-1 or analogs or active fragments thereof, or pharmaceutically acceptable salts thereof, for activation and mobilisation of MDSC for treating excessive dysregulated inflammatory diseases or conditions;
the use of an effective amount of inhibitors of SAA, CRP and/or CXCL-1 activity or a pharmaceutically acceptable salt thereof for reducing the frequency, accumulation and/or activity, and/or for depleting MDSC for treating diseases or conditions **characterised by** suppressed immune response;
the use of an effective amount of SAA, CRP and/or CXCL-1 or analogs or active fragments thereof or pharmaceutically acceptable salts thereof for suppressing inflammation in individuals afflicted with dysregulated inflammatory diseases or conditions;
a method for treatment of dysregulated inflammatory diseases or conditions, in particular, sepsis, by administering an effective amount of an active ingredient of SAA, CRP and/or CXCL-1 or analogs of active fragments thereof or pharmaceutically acceptable salts thereof to an individual in need thereof; and
a method for the treatment of malignancies in individuals by administering an effective amount of an inhibitor of SAA, CRP and/or CXCL-1 or a pharmaceutically acceptable salt thereof for inactivating, depleting or otherwise suppressing MDSC.

Delano, M.J., et al., 2007. J Exp Med. 204:1463-74.
Haile, L.A., et al., 2008. 135:871-81, 881 e1-5.
Hoechst, B., et al., 2008. Gastroenterology. 135:234-43.
Kim, K.D., et al., 2007. Nat Med. 13:1248-52 .
Klein, C., T. et al., 2005. J Clin Invest. 115:860-9.
Nagaraj, S., et al., 2007. Nat Med. 13:828-35 .
Nefedova, et al., 2004. J Immunol. 172:464-74.
Rebouissou, S., et al., 2009. Nature. 457:200-4 .
Sander, L., et al., 2009. J of Hepatology. 50: page S280
Sinha, P., et al., 2007. J Immunol. 179:9
Yang, L., et al., 2008. Cancer Cell. 13:23-35.
Young, et al., 1987. Cancer Res. 47:100-5.
Zhu, B., et al., 2007. J Immunol. 179:5228-37.

## Claims

1. Pharmaceutical composition containing an active ingredient, wherein the active ingredient is an effective amount of serum amyloid A(SAA) alone or active fragment thereof, or a pharmaceutically acceptable salt thereof for the use in the treatment of dysregulated inflammatory diseases or conditions by enhancing the activity of myeloid-derived suppressor cells (MDSC), wherein the dysregulated inflammatory diseases or conditions are autoinflammatory diseases or autoinflammatory diseases with autoimmune components or autoimmune diseases including IBD, rheumatoid arthritis, psoriasis, multiple sclerosis, transplant rejection, graft vs. host and host vs. graft disease, local and systemic inflammatory diseases, a systemic inflammatory response syndrome, sepsis, severe sepsis, septic shock, multi organ dysfunction syndrome, multiple organ failure syndrome and acute transplant rejection.

2. Pharmaceutical composition according to claim 1 containing an active ingredient, wherein the active ingredient is an effective amount of SAA or active fragment thereof, or a pharmaceutically acceptable salt thereof for use in the reduction, amelioration or termination of inflammatory responses or slow down the propagation or further escalation of inflammation or a disease associated with dysregulated inflammatory diseases or conditions by enhancing the activity of MDSC.

3. The pharmaceutical composition according to claim 1 or 2 wherein the active ingredient is an invariant peptide at position 33-43 of SAA.

4. The pharmaceutical composition according to any one of the preceding claims further comprising pharmaceutical additives or auxiliary substances and, preferably, a pharmaceutically acceptable carrier, excipient or diluent.

5. An effective amount of SAA alone or active fragments thereof or pharmaceutically acceptable salts thereof for use in treatment of sepsis.

## Patentansprüche

1. Pharmazeutische Zusammensetzung mit einem aktiven Inhaltsstoff, wobei der aktive Inhaltsstoff eine effektive Menge von SAA alleine oder ein aktives Fragment davon, oder ein pharmazeutisch akzeptables Salz davon ist, für die Verwendung in der Behandlung von dysregulierten inflammatorischen Erkrankungen oder Zuständen durch Verstärkung der Aktivität von Myeloidabgeleiteten Suppressorzellen (MDSC), wobei die dysregulierten inflammatorischen Erkrankungen oder Zustände autoinflammatorische Erkrankungen oder autoinflammatorische Erkrankungen mit Autoimmunkomponenten oder Autoimmunerkrankungen einschließlich IBD, rheumatische Arthritis, Psoriasis, Multiple Sklerose, Transplantatabstoßung, Transplantat-versus-Wirt-Reaktion oder Wirt-versus-Transplantat-Reaktion, lokale und systemische inflammatorische Erkrankungen, systemisches inflammatorisches Reaktionssyndrom, Sepsis, schwere Sepsis, septischer Schock, multiples Organfehlfunktionsversagen, multiples Organversagen und akute Transplantatabstoßung sind.

2. Pharmazeutische Zusammensetzung nach Anspruch 1, enthaltend einen aktiven Inhaltsstoff, wobei der aktive Inhaltsstoff eine effektive Menge von SAA allein oder ein aktives Fragment davon, oder ein pharmazeutisch akzeptables Salz davon ist, für die Verwendung in der Reduktion, Verbesserung oder Unterbrechung von inflammatorischen Antwortreaktionen oder Verlangsamung der Ausbreitung oder weiterer Eskalation der Entzündung oder einer Erkrankung assoziiert mit dysregulierten inflammatorischen Erkrankungen oder Zuständen durch Verstärkung der Aktivität der MDSC.

3. Pharmazeutische Zusammensetzung nach Anspruch 1 oder 2, wobei der aktive Inhaltsstoff ein invariantes Peptid an Position 33-43 von SAA ist.

4. Pharmazeutische Zusammensetzung nach einem der vorherigen Ansprüche, zusätzlich umfassend pharmazeutische Additive oder Hilfsstoffe und, vorzugsweise, einen pharmazeutisch akzeptablen Träger, Bindemittel oder Verdünner.

5. Eine effective Menge von SAA allein oder aktive Fragmente davon oder pharmazeutisch akzeptable Salze davon für die Verwendung bei der Behandlung von Sepsis.

## Revendications

1. La composition pharmaceutique contenant un ingrédient actif, dans lequel l'ingrédient actif est une quantité efficace de SAA seule ou un fragment biologiquement actif de celle-ci, ou un de ses sels pharmaceutiquement acceptables pour son usage dans le traitement des maladies ou conditions inflammatoires dérégulées en augmentant l'activité des cellules dérivées myéloïdes suppressives (MDSC), dans laquelle les maladies ou conditions inflammatoires dérégulées sont des maladies auto inflammatoires ou des maladies auto inflammatoires avec des composants auto-immunitaires ou des maladies auto-immunitaires incluant les MICI, arthrite rhumatoïde, psoriasis, sclérose en plaques, rejets de greffes, maladies du greffon contre l'hôte ou l'hôte contre le greffon, maladies inflammatoires locales et systémiques, syndrome de réponse inflammatoire systémique, septicémie, sepsis sévère et choc septique, dysfonctionnement des organes viscéraux, syndrome de défaillance multiviscérale et rejet aigu du greffon.

2. La composition pharmaceutique selon la revendication 1 contenant un ingrédient actif, dans lequel l'ingrédient actif est une quantité efficace de SAA seule ou un fragment biologiquement actif de celle-ci, ou un de ses sels pharmaceutiquement acceptables pour son usage dans la réduction, l'amélioration ou l'interruption des réactions inflammatoires ou le ralentissement de la propagation ou de l'aggravation de l'inflammation ou d'une maladie associée avec les maladies ou conditions inflammatoires dérégulées en augmentant l'activité des MDSC.

3. La composition pharmaceutique selon la revendication 1 ou 2 dans laquelle l'ingrédient actif est un peptide invariable aux positions 33-43 de SAA.

4. La composition pharmaceutique selon l'une quelconque des revendications 1 à 3 comprenant en plus des additifs pharmaceutiques ou des substances auxiliaires et, préférablement, un porteur pharmaceutiquement acceptable, excipient ou diluant.

5. Une quantité efficace de SAA seule ou des fragments actifs de celle-ci ou des sels pharmaceutiquement acceptables pour son usage dans le traitement de la septicémie.


### SEQUENCE LISTING

<110> RWTH Aachen
<120> Pharmaceutical composition for acute phase targeted treatment of acute and chronic dysregulated inflammatory diseases or conditions
<130> 4589-003
<160> 10
<170> PatentIn version 3.3
<210> 1
   <211> 5
   <212> PRT
   <213> artificial
<220>
   <223> peptide
<400> 1
   Tyr Ile Gly Ser Asp
   1               5
<210> 2
   <211> 5
   <212> PRT
   <213> artificial
<220>
   <223> peptide
<400> 2
   Tyr Ile Gly Ser Arg
   1               5
<210> 3
   <211> 1103
   <212> DNA
   <213> human CXCL1
<220>
   <221> CDS
   <222> (80)..(403)
<400> 3
   cacagagccc gggccgcagg cacctcctcg ccagctcttc cgctcctctc acagccgcca      60
   gacccgcctg ctgagcccc atg gcc cgc gct gct ctc tcc gcc gcc ccc agc      112
                        Met Ala Arg Ala Ala Leu Ser Ala Ala Pro Ser
                        1               5                   10
   aat ccc cgg ctc ctg cga gtg gca ctg ctg ctc ctg ctc ctg gta gcc      160
   Asn Pro Arg Leu Leu Arg Val Ala Leu Leu Leu Leu Leu Leu Val Ala
               15                  20                  25
   gct ggc cgg cgc gca gca gga gcg tcc gtg gcc act gaa ctg cgc tgc      208
   Ala Gly Arg Arg Ala Ala Gly Ala Ser Val Ala Thr Glu Leu Arg Cys
           30                  35                  40
   cag tgc ttg cag acc ctg cag gga att cac ccc aag aac atc caa agt      256
   Gln Cys Leu Gln Thr Leu Gln Gly Ile His Pro Lys Asn Ile Gln Ser
       45                  50                  55
   gtg aac gtg aag tcc ccc gga ccc cac tgc gcc caa acc gaa gtc ata      304
   Val Asn Val Lys Ser Pro Gly Pro His Cys Ala Gln Thr Glu Val Ile
   60                  65                  70                  75
   gcc aca ctc aag aat ggg cgg aaa gct tgc ctc aat cct gca tcc ccc      352
   Ala Thr Leu Lys Asn Gly Arg Lys Ala Cys Leu Asn Pro Ala Ser Pro
                   80                  85                  90
   ata gtt aag aaa atc atc gaa aag atg ctg aac agt gac aaa tcc aac      400
   Ile Val Lys Lys Ile Ile Glu Lys Met Leu Asn Ser Asp Lys Ser Asn
               95                  100                 105
   tga ccagaaggga ggaggaagct cactggtggc tgttcctgaa ggaggccctg           453
   cccttatagg aacagaagag gaaagagaga cacagctgca gaggccacct ggattgtgcc   513
   taatgtgttt gagcatcgct taggagaagt cttctattta tttatttatt cattagtttt    573
   gaagattcta tgttaatatt ttaggtgtaa aataattaag ggtatgatta actctacctg    633
   cacactgtcc tattatattc attctttttg aaatgtcaac cccaagttag ttcaatctgg     693
   attcatattt aatttgaagg tagaatgttt tcaaatgttc tccagtcatt atgttaatat    753
   ttctgaggag cctgcaacat gccagccact gtgatagagg ctggcggatc caagcaaatg    813
   gccaatgaga tcattgtgaa ggcaggggaa tgtatgtgca catctgtttt gtaactgttt    873
   agatgaatgt cagttgttat ttattgaaat gatttcacag tgtgtggtca acatttctca    933
   tgttgaaact ttaagaacta aaatgttcta aatatccctt ggacatttta tgtctttctt    993
   gtaaggcata ctgccttgtt taatggtagt tttacagtgt ttctggctta gaacaaaggg   1053
   gcttaattat tgatgttttc atagagaata taaaaataaa gcacttatag              1103
<210> 4
   <211> 107
   <212> PRT
   <213> human CXCL1
<400> 4
   Met Ala Arg Ala Ala Leu Ser Ala Ala Pro Ser Asn Pro Arg Leu Leu
   1               5                   10                  15
   Arg Val Ala Leu Leu Leu Leu Leu Leu Val Ala Ala Gly Arg Arg Ala
               20                  25                  30
   Ala Gly Ala Ser Val Ala Thr Glu Leu Arg Cys Gln Cys Leu Gln Thr
           35                  40                  45
   Leu Gln Gly Ile His Pro Lys Asn Ile Gln Ser Val Asn Val Lys Ser
       50                  55                  60
   Pro Gly Pro His Cys Ala Gln Thr Glu Val Ile Ala Thr Leu Lys Asn
   65                  70                  75                  80
   Gly Arg Lys Ala Cys Leu Asn Pro Ala Ser Pro Ile Val Lys Lys Ile
   85 90 95
   Ile Glu Lys Met Leu Asn Ser Asp Lys Ser Asn
   100 105
<210> 5
   <211> 716
   <212> DNA
   <213> human SAA1
<220>
   <221> CDS
   <222> (224)..(592)
<400> 5
   aggctcagta taaatagcag ccaccgctcc ctggcaggca gggacccgca gctcagctac      60
   agcacagatc aggtgaggag cacaccaagg agtgattttt aaaacttact ctgttttctc     120
   tttcccaaca agattatcat ttcctttaaa aaaaatagtt atcctggggc atacagccat     180
   accattctga aggtgtctta tctcctctga tctagagagc acc atg aag ctt ctc       235
                                                   Met Lys Leu Leu
                                                   1
   acg ggc ctg gtt ttc tgc tcc ttg gtc ctg ggt gtc agc agc cga agc       283
   Thr Gly Leu Val Phe Cys Ser Leu Val Leu Gly Val Ser Ser Arg Ser
   5                   10                  15                  20
   ttc ttt tcg ttc ctt ggc gag gct ttt gat ggg gct cgg gac atg tgg       331
   Phe Phe Ser Phe Leu Gly Glu Ala Phe Asp Gly Ala Arg Asp Met Trp
                   25                  30                  35
   aga gcc tac tct gac atg aga gaa gcc aat tac atc ggc tca gac aaa       379
   Arg Ala Tyr Ser Asp Met Arg Glu Ala Asn Tyr Ile Gly Ser Asp Lys
               40                  45                  50
   tac ttc cat gct cgg ggg aac tat gat gct gcc aaa agg gga cct ggg       427
   Tyr Phe His Ala Arg Gly Asn Tyr Asp Ala Ala Lys Arg Gly Pro Gly
           55                  60                  65
   ggt gcc tgg gct gca gaa gtg atc agc gat gcc aga gag aat atc cag       475
   Gly Ala Trp Ala Ala Glu Val Ile Ser Asp Ala Arg Glu Asn Ile Gln
       70                  75                  80
   aga ttc ttt ggc cat ggt gcg gag gac tcg ctg gct gat cag gct gcc       523
   Arg Phe Phe Gly His Gly Ala Glu Asp Ser Leu Ala Asp Gln Ala Ala
   85                  90                  95                  100
   aat gaa tgg ggc agg agt ggc aaa gac ccc aat cac ttc cga cct gct       571
   Asn Glu Trp Gly Arg Ser Gly Lys Asp Pro Asn His Phe Arg Pro Ala
                   105                 110                 115
   ggc ctg cct gag aaa tac tga gcttcctctt cactctgctc tcaggagatc          622
   Gly Leu Pro Glu Lys Tyr
               120
   tggctgtgag gccctcaggg cagggataca aagcggggag agggtacaca atgggtatct     682
   aataaatact taagaggtgg aatttgtgga aact                                 716
<210> 6
   <211> 122
   <212> PRT
   <213> human SAA1 saa_ep.ST25.txt
<400> 6
   Met Lys Leu Leu Thr Gly Leu Val Phe Cys Ser Leu Val Leu Gly Val
   1               5                   10                  15
   Ser Ser Arg Ser Phe Phe Ser Phe Leu Gly Glu Ala Phe Asp Gly Ala
               20                  25                  30
   Arg Asp Met Trp Arg Ala Tyr Ser Asp Met Arg Glu Ala Asn Tyr Ile
           35                  40                  45
   Gly Ser Asp Lys Tyr Phe His Ala Arg Gly Asn Tyr Asp Ala Ala Lys
       50                  55                  60
   Arg Gly Pro Gly Gly Ala Trp Ala Ala Glu Val Ile Ser Asp Ala Arg
   65                  70                  75                  80
   Glu Asn Ile Gln Arg Phe Phe Gly His Gly Ala Glu Asp Ser Leu Ala
                   85                  90                  95
   Asp Gln Ala Ala Asn Glu Trp Gly Arg Ser Gly Lys Asp Pro Asn His
               100                 105                 110
   Phe Arg Pro Ala Gly Leu Pro Glu Lys Tyr
           115                 120
<210> 7
   <211> 545
   <212> DNA
   <213> human SAA2
<220>
   <221> CD5
   <222> (38)..(406)
<400> 7
   agggacccgc agctcagcta cagcacagat cagcacc atg aag ctt ctc acg ggc      55
                                            Met Lys Leu Leu Thr Gly
                                            1               5
   ctg gtt ttc tgc tcc ttg gtc ctg agt gtc agc agc cga agc ttc ttt      103
   Leu Val Phe Cys Ser Leu Val Leu Ser Val Ser Ser Arg Ser Phe Phe
               10                  15                  20
   tcg ttc ctt ggc gag gct ttt gat ggg gct cgg gac atg tgg aga gcc      151
   Ser Phe Leu Gly Glu Ala Phe Asp Gly Ala Arg Asp Met Trp Arg Ala
           25                  30                  35
   tac tct gac atg aga gaa gcc aat tac atc ggc tca gac aaa tac ttc      199
   Tyr Ser Asp Met Arg Glu Ala Asn Tyr Ile Gly Ser Asp Lys Tyr Phe
       40                  45                  50
   cat gct cgg ggg aac tat gat gct gcc aaa agg gga cct ggg ggt gcc      247
   His Ala Arg Gly Asn Tyr Asp Ala Ala Lys Arg Gly Pro Gly Gly Ala
   55                  60                  65                  70
   tgg gct gca gaa gtg atc agc aat gcc aga gag aat atc cag aga ctc      295
   Trp Ala Ala Glu Val Ile Ser Asn Ala Arg Glu Asn Ile Gln Arg Leu
                   75                  80                  85
   aca ggc cgt ggt gcg gag gac tcg ctg gcc gat cag gct gcc aat aaa      343
   Thr Gly Arg Gly Ala Glu Asp Ser Leu Ala Asp Gln Ala Ala Asn Lys
               90                  95                  100
   tgg ggc agg agt ggc aga gac ccc aat cac ttc cga cct gct ggc ctg      391
   Trp Gly Arg Ser Gly Arg Asp Pro Asn His Phe Arg Pro Ala Gly Leu
           105                 110                 115
   cct gag aaa tac tga gcttcctctt cactctgctc tcaggagacc tggctatgag      446
   Pro Glu Lys Tyr
       120
   gccctcgggg cagggataca aagttagtga ggtctatgtc cagagaagct gagatatggc    506
   atataatagg catctaataa atgcttaaga ggtggaaaa                           545
<210> 8
   <211> 122
   <212> PRT
   <213> human SAA2
<400> 8
   Met Lys Leu Leu Thr Gly Leu Val Phe Cys Ser Leu Val Leu Ser Val
   1               5                   10                  15
   Ser Ser Arg Ser Phe Phe Ser Phe Leu Gly Glu Ala Phe Asp Gly Ala
               20                  25                  30
   Arg Asp Met Trp Arg Ala Tyr Ser Asp Met Arg Glu Ala Asn Tyr Ile
           35                  40                  45
   Gly Ser Asp Lys Tyr Phe His Ala Arg Gly Asn Tyr Asp Ala Ala Lys
       50                  55                  60
   Arg Gly Pro Gly Gly Ala Trp Ala Ala Glu Val Ile Ser Asn Ala Arg
   65                  70                  75                  80
   Glu Asn Ile Gln Arg Leu Thr Gly Arg Gly Ala Glu Asp Ser Leu Ala
                   85                  90                  95
   Asp Gln Ala Ala Asn Lys Trp Gly Arg Ser Gly Arg Asp Pro Asn His
               100                 105                 110
   Phe Arg Pro Ala Gly Leu Pro Glu Lys Tyr
           115                 120
<210> 9
   <211> 2024
   <212> DNA
   <213> human CRP
<220>
   <221> CD5
   <222> (105)..(779)
<400> 9
   aaggcaagag atctaggact tctagcccct gaactttcag ccgaatacat cttttccaaa      60
   ggagtgaatt caggcccttg tatcactggc agcaggacgt gacc atg gag aag ctg      116
                                                    Met Glu Lys Leu
                                                    1
   ttg tgt ttc ttg gtc ttg acc agc ctc tct cat gct ttt ggc cag aca      164
   Leu Cys Phe Leu Val Leu Thr Ser Leu Ser His Ala Phe Gly Gln Thr
   5                   10                  15                  20
   gac atg tcg agg aag gct ttt gtg ttt ccc aaa gag tcg gat act tcc      212
   Asp Met Ser Arg Lys Ala Phe Val Phe Pro Lys Glu Ser Asp Thr Ser
                   25                  30                  35
   tat gta tcc ctc aaa gca ccg tta acg aag cct ctc aaa gcc ttc act      260
   Tyr Val Ser Leu Lys Ala Pro Leu Thr Lys Pro Leu Lys Ala Phe Thr
               40                  45                  50
   gtg tgc ctc cac ttc tac acg gaa ctg tcc tcg acc cgt ggg tac agt      308
   Val Cys Leu His Phe Tyr Thr Glu Leu Ser Ser Thr Arg Gly Tyr Ser
           55                  60                  65
   att ttc tcg tat gcc acc aag aga caa gac aat gag att ctc ata ttt      356
   Ile Phe Ser Tyr Ala Thr Lys Arg Gln Asp Asn Glu Ile Leu Ile Phe
       70                  75                  80
   tgg tct aag gat ata gga tac agt ttt aca gtg ggt ggg tct gaa ata      404
   Trp Ser Lys Asp Ile Gly Tyr Ser Phe Thr Val Gly Gly Ser Glu Ile
   85                  90                  95                  100
   tta ttc gag gtt cct gaa gtc aca gta gct cca gta cac att tgt aca      452
   Leu Phe Glu Val Pro Glu Val Thr Val Ala Pro Val His Ile Cys Thr
                   105                 110                 115
   agc tgg gag tcc gcc tca ggg atc gtg gag ttc tgg gta gat ggg aag      500
   Ser Trp Glu Ser Ala Ser Gly Ile Val Glu Phe Trp Val Asp Gly Lys
               120                 125                 130
   ccc agg gtg agg aag agt ctg aag aag gga tac act gtg ggg gca gaa      548
   Pro Arg Val Arg Lys Ser Leu Lys Lys Gly Tyr Thr Val Gly Ala Glu
           135                 140                 145
   gca agc atc atc ttg ggg cag gag cag gat tcc ttc ggt ggg aac ttt      596
   Ala Ser Ile Ile Leu Gly Gln Glu Gln Asp Ser Phe Gly Gly Asn Phe
       150                 155                 160
   gaa gga agc cag tcc ctg gtg gga gac att gga aat gtg aac atg tgg      644
   Glu Gly Ser Gln Ser Leu Val Gly Asp Ile Gly Asn Val Asn Met Trp
   165                 170                 175                 180
   gac ttt gtg ctg tca cca gat gag att aac acc atc tat ctt ggc ggg      692
   Asp Phe Val Leu Ser Pro Asp Glu Ile Asn Thr Ile Tyr Leu Gly Gly
                   185                 190                 195
   ccc ttc agt cct aat gtc ctg aac tgg cgg gca ctg aag tat gaa gtg      740
   Pro Phe Ser Pro Asn Val Leu Asn Trp Arg Ala Leu Lys Tyr Glu Val
               200                 205                 210
   caa ggc gaa gtg ttc acc aaa ccc cag ctg tgg ccc tga ggcccagctg      789
   Gln Gly Glu Val Phe Thr Lys Pro Gln Leu Trp Pro
           215                 220
   tgggtcctga aggtacctcc cggtttttta caccgcatgg gccccacgtc tctgtctctg      849
   gtacctcccg cttttttaca ctgcatggtt cccacgtctc tgtctctggg cctttgttcc      909
   cctatatgca ttgcaggcct gctccaccct cctcagcgcc tgagaatgga ggtaaagtgt    969
   ctggtctggg agctcgttaa ctatgctggg aaacggtcca aaagaatcag aatttgaggt   1029
   gttttgtttt catttttatt tcaagttgga cagatcttgg agataatttc ttacctcaca   1089
   tagatgagaa aactaacacc cagaaaggag aaatgatgtt ataaaaaact cataaggcaa   1149
   gagctgagaa ggaagcgctg atcttctatt taattcccca cccatgaccc ccagaaagca   1209
   ggagggcatt gcccacattc acagggctct tcagtctcag aatcaggaca ctggccaggt   1269
   gtctggtttg ggtccagagt gctcatcatc atgtcataga actgctgggc ccaggtctcc   1329
   tgaaatggga agcccagcaa taccacgcag tccctccact ttctcaaagc acactggaaa   1389
   ggccattaga attgccccag cagagcagat ctgctttttt tccagagcaa aatgaagcac   1449
   taggtataaa tatgttgtta ctgccaagaa cttaaatgac tggtttttgt ttgcttgcag   1509
   tgctttctta attttatggc tcttctggga aactcctccc cttttccaca cgaaccttgt   1569
   ggggctgtga attctttctt catccccgca ttcccaatat acccaggcca caagagtgga   1629
   cgtgaaccac agggtgtcct gtcagaggag cccatctccc atctccccag ctccctatct   1689
   ggaggatagt tggatagtta cgtgttccta gcaggaccaa ctacagtctt cccaaggatt   1749
   gagttatgga ctttgggagt gagacatctt cttgctgctg gatttccaag ctgagaggac   1809
   gtgaacctgg gaccaccagt agccatcttg tttgccacat ggagagagac tgtgaggaca   1869
   gaagccaaac tggaagtgga ggagccaagg gattgacaaa caacagagcc ttgaccacgt   1929
   ggagtctctg aatcagcctt gtctggaacc agatctacac ctggactgcc caggtctata   1989
   agccaataaa gcccctgttt acttgaaaaa aaaaa                              2024
<210> 10
   <211> 224
   <212> PRT
   <213> human CRP
<400> 10
   Met Glu Lys Leu Leu Cys Phe Leu Val Leu Thr Ser Leu Ser His Ala
   1               5                   10                  15
   Phe Gly Gln Thr Asp Met Ser Arg Lys Ala Phe Val Phe Pro Lys Glu
               20                  25                  30
   Ser Asp Thr Ser Tyr Val Ser Leu Lys Ala Pro Leu Thr Lys Pro Leu
           35                  40                  45
   Lys Ala Phe Thr Val Cys Leu His Phe Tyr Thr Glu Leu Ser Ser Thr
       50                  55                  60
   Arg Gly Tyr Ser Ile Phe Ser Tyr Ala Thr Lys Arg Gln Asp Asn Glu
   65                  70                  75                  80
   Ile Leu Ile Phe Trp Ser Lys Asp Ile Gly Tyr Ser Phe Thr Val Gly
                   85                  90                  95
   Gly Ser Glu Ile Leu Phe Glu Val Pro Glu Val Thr Val Ala Pro Val
   100 105 110
   His Ile Cys Thr Ser Trp Glu Ser Ala Ser Gly Ile Val Glu Phe Trp
   115 120 125
   Val Asp Gly Lys Pro Arg Val Arg Lys Ser Leu Lys Lys Gly Tyr Thr
   130 135 140
   Val Gly Ala Glu Ala Ser Ile Ile Leu Gly Gln Glu Gln Asp Ser Phe
   145 150 155 160
   Gly Gly Asn Phe Glu Gly Ser Gln Ser Leu Val Gly Asp Ile Gly Asn
   165 170 175
   Val Asn Met Trp Asp Phe Val Leu Ser Pro Asp Glu Ile Asn Thr Ile
   180 185 190
   Tyr Leu Gly Gly Pro Phe Ser Pro Asn Val Leu Asn Trp Arg Ala Leu
   195 200 205
   Lys Tyr Glu Val Gln Gly Glu Val Phe Thr Lys Pro Gln Leu Trp Pro
   210 215 220